# EUROPEAN PATENT SPECIFICATION

(11) **EP 0 695 166 B1**
(45) Date of publication and mention of the grant of the patent: **24.10.2001**
(21) Application number: 94913749.1
(22) Date of filing: 29.04.1994
(51) Int. Cl.: A61K 7/46

(54) **PERFUME COMPOSITION**
PARFUMZUSAMMENSETZUNG
COMPOSITION DE PARFUM

(30) Priority: 30.04.1993 GB 9308953
(43) Date of publication of application: 07.02.1996
(73) Proprietor: QUEST INTERNATIONAL NEDERLAND BV, 1411 GP Naarden (NL)
(72) Inventor: BEHAN, John Martin, Kent TN25 4EB (GB); CLEMENTS, Christopher Francis, Folkestone Kent CT20 2LB (GB); MARTIN, John Robert, Merseyside L43 0RA (GB); PERRING, Keith Douglas, Kent TN24 8HS (GB)
(74) Representative: Ford, Michael Frederick
(86) International application number: GB9400935
(87) International publication number: WO9424999

(56) References cited:
- EP-A- 0 003 171
- EP-A- 0 003 172
- EP-A- 0 005 618
- EP-A- 0 147 191
- EP-A- 0 545 556
- US-A- 4 134 838

## Description

This invention relates to perfume compositions, that is to say compositions of fragrance materials, to the use of such perfume compositions to give a deodorant effect, and to fabric conditioning compositions containing such perfume compositions.

EP-B-3172, US-A-4304679, US-A-4322308, US-A-4278658, US-A-4134838, US-A-4288341, US-A-4289641 and US-A-4663068 all describe perfume compositions which exhibit a deodorant action, (i.e. inhibit development of human body malodour). US-A-4134838 teaches that such perfume compositions may be included in a fabric conditioning product used to soften fabrics during rinsing or drying.

A difficulty with the perfume compositions disclosed in these documents is that they include components which frequently give them strong, powerful odours which are difficult for the perfumer to blend out and which can limit the usefulness of the compositions when used to perfume some other product such as a detergent composition, fabric conditioner or personal care product. This has created a necessity for compromise between deodorant efficacy and acceptability as a fragrance.

We have now found that deodorant perfumes can be made by the use of materials from a combination of certain specified categories. The use of materials from this combination of categories makes it possible to obtain good deodorant performance and widely acceptable fragrance.

Our copending, European application published as EP 545556 on 9 June 1993 describes deodorant perfumes made from specified categories of materials which always include at least 0.2% by weight of the perfume compositions of one or more ethers of general formula

R¹OR²

in which the groups R¹ and R² are connected only through the ether oxygen atom, and are aliphatic or aromatic groups such that the ether has a molecular weight of 150 to 200.

By contrast the perfume compositions of this invention do not require such ethers as essential ingredients, although they may optionally be present.

Broadly, the present invention provides a perfume composition in which at least 30% by weight of the composition is constituted by materials from the categories of materials set out below:
at least 7%, preferably at least 10% and generally not more than 50% by weight of the perfume composition of one or more aromatic methyl ketones of general formula 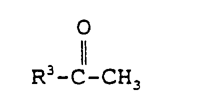 in which R³ is an aromatic group such that the molecular weight of the ketone is from 170 to 300;
at least 5% and generally not more than 70% by weight of the perfume composition of one or more ingredients selected from alcohols of general formula:

   R⁴OH

   acetates of general formula

   CH₃CO₂R⁵

   and propionates of general formula

   C₂H₅CO₂R⁵

   in which R⁴ is an aliphatic group, optionally containing not more than one olefinic double bond, and optionally bearing an aromatic substituent group, such that the molecular weight of an alcohol R⁴OH is in the range 130 to 180 and R⁵ is an aliphatic group optionally containing not more than one olefinic double bond, and optionally bearing an aromatic substituent group, such that the molecular weight of an acetate or propionate ester is in the range 180 to 210;
at least 3% and generally not more than 60% by weight of the perfume composition of one or more i salicylates of general formula 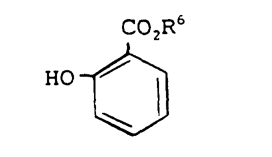 in which R⁶ is an aliphatic group, optionally containing not more than one olefinic double bond, and optionally bearing an aromatic substituent group, such that the molecular weight of the salicylate is in the range 190 to 230;
all the percentages being by weight of the whole perfume composition.

It is preferred that the perfume compositions contain at least 4% of one or more of the above alcohols of formula R⁴OH and at least 5% of one or more of the above esters of formula CH₃CO₂R⁵ or C₂H₅CO₂R⁵.

As mentioned above the perfume compositions of this invention may include at least 0.05% of one or more ethers of the general formula

R¹OR²

in which the groups R¹ and R² are connected only through the ether oxygen atom, and are aliphatic or aromatic groups such that the ether has a molecular weight of 150 to 200.

It should be appreciated that the present invention includes perfume compositions in which the content of such ethers is not more than 0.19% by weight of the composition and also includes compositions containing at least 0.2% of such ethers, but where the total content of the said ethers, ketones, alcohols, acetates, propionates and salicylates lies in a range from 30 to 49.9% by weight of the perfume composition.

In one aspect this invention provides a fabric conditioning product for use in the treatment of fabrics, during rinsing or drying which composition contains a fabric softening agent together with a perfume composition as set forth above.

In another aspect this invention provides the use, as a deodorant, of a perfume composition as set forth above.

The categories of perfume materials referred to above will now be reviewed in turn.

### Aromatic methyl ketones

The group R³ in the formula 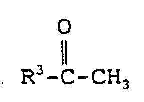 given above can contain up to approximately 18 carbon atoms and will usually contain at least 9. Examples of suitable ketones are:-
Alpha or beta methyl naphthyl ketone;
Musk ketone, which is a trivial name for 4-tert-butyl-3,5-dinitro-2,6-dimethyl acetophenone;
1,1,2,4,4,7-Hexamethyl-6-acetyl-1,2,3,4-tetrahydronaphthalene, available under the trademark "TONALID";
5-Acetyl-1,1,2,3,3,6-hexamethylindane, available under the trademark "PHANTOLIDE";
4-Acetyl-6-tert-butyl-1,1-dimethylindane, available under the trademark "CELESTOLIDE";
6-Acetyl-1-isopropyl-2,3,3,5-tetramethylindane, available under the trademark "TRASEOLIDE";
1,1,4,4-Tetramethyl-6-acetyl-7-ethyl-1,2,3,4-tetrahydronaphthalene, available under the trademark "VERSALIDE".

The amount of each ketone, if more than one is present, will desirably be at least 1% or at least 2% by weight of the perfume composition. The total amount of these ketones may extend up to 35% or even beyond up to 50% by weight of the perfume composition. Possibly, however, the amount does not exceed 24%, 20% or 18% by weight of the perfume composition and may lie in a range from 7 to 15%.

### Alcohols

The group R⁴ in the formula R⁴OH given above is aliphatic but may have an aromatic substituent. Olefinic unsaturation may be present to the extent of one double bond, but may be entirely absent. Aliphatic groups are therefore alkyl, alkenyl, cycloalkyl and cycloalkenyl, optionally bearing an aromatic substituent group.

The stated molecular weight range of 130 to 180 permits up to 11 carbon atoms in the group R⁴. Usually there will be at least 8. Examples of suitable alcohols are:-
Cinnamic alcohol
Citronellol
Decanol
Dihydromyrcenol
Dimethylheptanol
Dimethyloctanol
Dimethyl benzyl carbinol
Isononanol
Isoborneol
4-isopropyl cyclohexanol
4-isopropyl cyclohexyl methanol
Isopulegol
Menthol
Myrtenol
Nonanol
Octanol
para-menthan-7-ol
2-tert-butylcyclohexanol
4-tert-butylcyclohexanol
3-methyl-5-phenyl pentanol, available under the trademark
"PHENOXANOL"
2-Phenylpropanol
3-Phenylpropanol
9-Decen-1-ol, available under the trademark "ROSALVA" alpha-Terpineol
beta-Terpineol
Tetrahydrogeraniol
Tetrahydrolinalol
3,5,5-Trimethylcyclohexanol
Undecanol
10-Undecen-1-ol.

The amount of individual alcohols is preferably at least 1% or at least 2% by weight of the perfume composition. The total amount of alcohol is preferably at least 4%, more preferably at least 5% but will generally not exceed 50% by weight of the perfume composition. From 7% to 30%, especially 7% to 20%, is preferred.

### Esters

These esters are acetates and propionates. Like the group R⁴, discussed above, the group R⁵ in the formula CH₃CO₂R⁵ and C₂H₅CO₂R⁵ given above is aliphatic, possibly with an aromatic substituent, and with no more than one olefinic double bond, if any.

The molecular weight range permits propionates in which R⁵ has up to 9 carbon atoms, and acetates in which R⁵ has up to 10 carbon atoms.

Examples of suitable esters are:-
3a,4,5,6,7,7a-hexahydro-4,7-methano-1(3 )H-inden-6-yl propanoate, available under the trademark "FLOROCYCLENE"; 3-acetoxymethyl-4,7,7-trimethylbicyclo[4.1.0]-hept-2-ene, available under the trademark "FORESTONE";
3a,4,5,6,7,7a-hexahydro-4,7-methano-1(3)H-inden-6-yl acetate, available under the trademark "JASMACYCLENE";
Bornyl acetate
Cinnamyl propionate
Citronellyl acetate
Decyl acetate
Dihydroterpinyl acetate
Dimethyl benzyl carbinyl acetate
3,5,5-trimethylhexyl acetate, available as "Inonyl acetate"
Isobornyl acetate
Isopulegol acetate
Menthyl acetate
Myrtenyl acetate
Myrtenyl propionate
Nonyl acetate
Terpinyl acetate
Terpinyl propionate
2-tert-butylcyclohexyl acetate
4-tert-butylcyclohexyl acetate
Tetrahydrogeranyl acetate
Tetrahydrolinalyl acetate
10-Undecenyl acetate.

The amounts of individual esters preferably are at least 1% or at least 2%. The total amount of esters can range up to as much as 40% by weight of the perfume composition or more. 5% to 30% is preferred. The amount may possibly be at least 10% by weight.

### Salicylates

In the formula 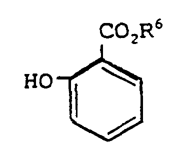 given above, the group R⁶, like the groups R⁴ and R⁵ mentioned above, is aliphatic, possibly with an aromatic substituent, and either without olefinic unsaturation, or with one double bond at most. The requirement as to molecular weight permits groups R⁶ of up to 7 carbon atoms. Examples of suitable salicylates are:-
Amyl salicylate
Benzyl salicylate
Butyl salicylate
cis-3-hexenyl salicylate
Cyclohexyl salicylate
Hexyl salicylate
Isoamyl salicylate
Isobutyl salicylate.

Salicylates can be used in large amounts, such as up to 50 or 60% by weight of the composition. At least 5%, may be preferred, and especially 5% to 20% by weight.

It may be preferred that the total amount of the said ketones and salicylates is not more than 35% by weight of the perfume composition.

### Ethers

These ethers are non-cyclic, in the sense that the ether oxygen atom is not part of a ring, although the groups R¹ and R² in the formula R¹OR² given above may themselves incorporate rings. Each of these groups may be aliphatic or aromatic e.g. alkyl, cycloalkyl, alkenyl, cycloalkenyl, phenyl, naphthyl, aryl substituted aliphatic or alkyl substituted aromatic. Preferably neither group contains more than one olefinic double bond.

The molecular weight range approximately corresponds to ethers containing up to about 13 or 14 carbon atoms in all. There will usually be at least 9 carbon atoms, depending however on any side chains present.

Examples of ethers in this category are:-
Phenylethyl isoamyl ether, available under the trademark "ANTHER";
Phenylethyl n-butyl ether;
Benzyl isoamyl ether;
Dihydroanethole, which is 4-propylanisole, more properly known as methyl 4-propylphenyl ether;
Diphenyl oxide;
p-tert butylphenyl methyl ether, available under the trademark "EQUINOL";
Ethyl naphthyl ether, also known under the trademark "NEROLIN";
Methyl naphthyl ether, available under the trademark "YARA".

The last five of the above ethers have at least one aromatic group which is phenyl, naphthyl or substituted phenyl or naphthyl.

Many of these ethers are effective when used in rather small amounts. Generally if more than one ether is present, each ether will be present in an amount of at least 0.1% by weight of the perfume composition. It will generally be desirable that the total amount of these ethers does not exceed 20% by weight of the perfume composition and possibly does not exceed 10% if a mixed aliphatic aromatic ether is present. A quantity of not over 6% is preferred, for methyl naphthyl ether and/or ethyl naphthyl ether. The total of all ethers in this category may well not exceed 6%.

A material may have a structure such that it can be placed in more than one of the above categories. If so, the material should be placed in only a single category.

Preferably, however, the assignment of materials to categories is carried out in such a way that any material which is simultaneously more than one of ether, ester, alcohol or ketone is first classified as an ester, alcohol, ketone or ether in that order of priority and then either attributed to the appropriate category if the material satisfies the requirements for that category, or else excluded from all categories.

The effect of this preferred approach is that the Category of alcohols shall not then include any material which is an ester (regardless of whether it is an acetate, propionate or some other ester). The category of ketones shall not include any material which is an ester or contains a hydroxyl group. The category of ethers shall not include any material which is an ester, or contains a hydroxyl or keto group.

For example on this basis a material which was both an ether and an alcohol would be treated as an alcohol and if it satisfied the above definition of category alcohols R⁴OH, or else excluded entirely. Similarly an ester which was not of formula:

CH₃CO₂R⁵ or C₂H₅CO₂R⁵

would not be placed in any category.

As a practical matter, available salicylates do not have other functionality. However, it should be the case that the categories of ethers, ketones, acetates and propionates do not include any material which is a salicylate.

### Further materials

The perfume compositions of this invention may include other materials in addition to those in the above categories. These may include at least 2% by weight of the perfume composition falling within a sixth category of specified materials which are not all structurally related. Members of this further category are:-
1) Aldehydes of formula R⁷CHO having molecular weight 180-220 in which R⁷ is aliphatic or aryl-aliphatic, like R⁴ and R⁵. Especially envisaged are hexyl cinnamic aldehyde, and 2-methyl-3(para-t-butylphenyl)propionaldehyde which is available under the trademark "LILIAL".
2) 1,3,4,6,7,8-hexahydro-4,6,6,7,8,8-hexamethylcyclopenta-2-benzopyran, available under the trademark "GALAXOLIDE".

It may be the case that the Categories if ethers, alcohols, acetates and propionates do not include any material which contains an aldehyde group.

Some compositions exemplified in prior documents have included natural essential oils. Many such oils contain substantial amounts of terpenes and terpene aldehydes. These natural oils tend to give strong odours and preferably are not used in amounts greater than 25%, better not greater than 10% by weight of the composition.

The perfume compositions set forth above are generally utilised incorporating them into a product which is applied to human skin, e.g. an underarm deodorant or a soap or other personal washing product, or into a product which is used in the laundering of fabrics which are subsequently worn.

The concentration of perfume in a product will generally lie in the range from 0.01% to 10% by weight, and in many instances will be not more than 5% or even 3% by weight.

A fabric washing composition will typically contain 2% to 50% by weight of detergent active and 5% to 80% by weight of detergency builder. A preferred amount of perfume for use in many fabric washing products is from 0.1% to 1.5% by weight.

Bars and other detergent compositions for personal washing will generally include at least 5% by weight of soap or non-soap detergent active. A product in bar form may contain 20% to 95% of soap or non-soap detergent active. A product in liquid form will generally contain 5% to 50% by weight of detergent active.

Products for personal care, such as talcs and underarm products will contain the perfume composition in a cosmetically acceptable vehicle or carrier.

The amount of perfume used in soap and/or non-soap detergent bars for personal washing is preferably 0.2 to 2% by weight of the bars, especially 0.4 to 1%. The amount used in personal body deodorants is preferably 0.1 to 3% especially 0.4 to 1% by weight of the deodorant product. It is possible to envisage products with higher proportions of perfume although still a minority proportion of the product, e.g. up to 25% by weight.

The perfume compositions set forth above are particularly intended to be incorporated in fabric conditioning products used during rinsing or drying of fabrics, notably to enhance softness of fabrics.

Fabric conditioning products are used during the rinsing and/or drying of fabrics to confer a benefit to the fabrics and to the user's perception of the fabrics.

The main benefits delivered by such products are softness, fragrance and anti-static. This invention is particularly concerned with products which provide softness. The perfume of this invention will then provide fragrance.

A fabric softening product contains at least one softening agent which functions to give the fabric a softer handle. Frequently such agents also provide an anti-static benefit. Usually such agents are cationic, nonionic, amphoteric or zwitterionic materials,

Many fabric softening products take the form of compositions intended to be added to rinse water. The fabric softening agents are then materials with low solubility in water, and which deposit on the fabrics. Typically the solubility in acidified water at 20°C is less than 10g/litre, preferably less than lg/litre. When added to rinse water such materials form a dispersed phase which is then able to deposit on fabrics which are being rinsed in the water.

An alternative type of fabric softening product is an article such as an impregnated sheet or a porous sachet which is placed in a tumble dryer. The fabric softening agent is arranged to melt at the temperature in the dryer. It then transfers from the article to the fabrics in the dryer.

For such products the fabric softening agents may be similar to those used in a composition which is added to rinse water.

Many commercially important fabric softening agents are organic compounds containing quaternary nitrogen and at least one carbon chain of 6 to 30 carbon atoms, e.g. in an alkyl, alkenyl or aryl substituted alkyl or alkenyl group with at least six aliphatic carbon atoms.

Other fabric softening agents are the corresponding tertiary amines and imidazolines, other aliphatic alcohols, esters, amines or carboxylic acids incorporating a C8 to C30 alkyl, alkenyl or acyl group, including esters of sorbitan and esters of polyhydric alcohols, mineral oils, polyols such as polyethylene glycol, and also clays. Some of these materials may be used jointly with others rather than alone. Another class of materials used as an adjunct to a fabric softening agent is hydrophobically modified cellulose ethers.

Some specific instances of fabric softening agents are:

### 1) Acyclic quaternary ammonium compounds of the formula (I)

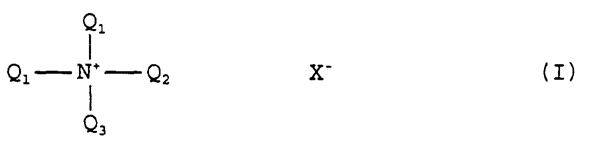

wherein each Q₁ is a hydrocarbyl group containing from 15 to 22 carbon atoms, Q₂ is a saturated alkyl or hydroxy alkyl group containing from 1 to 4 carbon atoms, Q₃ may be as defined for Q₁ or Q₂ or may be phenyl and X⁻ is an anion preferably selected from halide, methyl sulphate and ethyl sulphate radicals.

Throughout this discussion of fabric softening agents the expression hydrocarbyl group refers to alkyl or alkenyl groups optionally substituted or interrupted by functional groups such as -OH, -O-, CONH, -COO-, etc.

Representative examples of these quaternary softeners include ditallow dimethyl ammonium chloride; ditallow dimethyl ammonium methyl sulphate; dihexadecyl dimethyl ammonium chloride; di(hydrogenated tallow) dimethyl ammonium methyl sulphate or chloride; di(coconut)dimethyl ammonium chloride dihexadecy diethyl ammonium chloride; dibehenyl dimethyl ammonium chloride.

Ditallow dimethyl ammonium chloride, di(hydrogenated tallow) dimethyl ammonium chloride, di(coconut) dimethyl ammonium chloride and di(coconut) dimethyl ammonium methosulphate are preferred.

Examples of commercially available materials in this class are ARQUAD 2C, ARQUAD 2HT, ARQUAD 2T (all Ex Akzo Chemie), PRAPAGEN WK, PRAPAGEN WKT, DODIGEN 1828 (all Hoechst). QUERTON 4BG, QUERTON 442 (all Keno Gard), AMMONYX KP, AMMONYX SKD (all Mill Master-Onyx), SYNPROLAM FS (ICI).

### 2) Alkoxylated Polyamines

Alkoxylated polyamines of general formula (II) have been disclosed in EP 406 A1 (Procter)

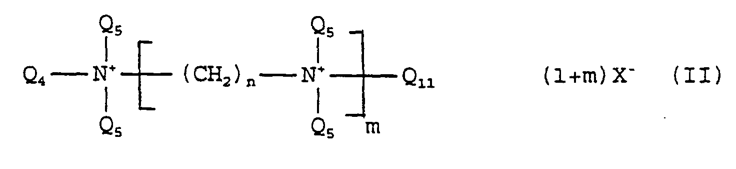

Each Q₄ is a hydrocarbyl group containing from 10 to 30 carbon atoms. The Q₅ groups may be the same or different each representing hydrogen, (-C₂H₄O)ₚH, (C₃H₆O)_{q}H, (C₂H₄O)ₚ, (C₃H₆O)_{q},H, an alkyl group containing from 1 to 3 carbon atoms or the group (CH₂)ₙ,N(Q₅)₂; n and n' are each an integer from 2 to 6, m is an integer from 1 to 5 and p, q and (p' + q') may be numbers such that (p + q + p' + q') does not exceed 25. X⁻ is an anion.

Alkoxylated polyamines suitable for use herein include N-tallowyl, NN'N'-tris (2 hydroxyethyl)-1,3-propane diamine di-hydro chloride; N-cocyl N,N,N',N' pentamethyl-1,3 propane diammonium dichloride or dimethosulphate; N-stearyl N,N',N' tris (2-hydroxyethyl) N,N1'dimethyl-1,3 propanediammonium dimethyl sulphate; N-palmityl N,N',N'tris (3-hydroxypropyl)-1,3-propanediammonium dihydrobromide; N-tallowyl N-(3 aminopropyl) -1,3-propanediamine trihydrochloride.

### 3. Diamido Quaternary Ammonium Salts

Diamido quaternary salts of general formula (III) are also known to be useful as fabric softening agents.

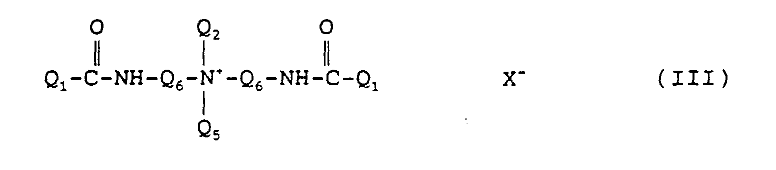

Q₆ is a divalent alkylene group containing from 1 to 3 carbon atoms. Q₁, Q₂, Q₅ and X⁻ are as defined previously.

Examples of suitable materials are methylbis (tallowamidoethyl)(2-hydroxyethyl) ammonium methyl sulphate and methyl bis (hydrogenated tallowamido ethyl)(2 hydroxyethyl) ammonium methyl sulphate. These materials are available from Sherex Chem Co under trade names VARISOFT 222 and VARISOFT 110 respectively and under the trade name ACCOSOFT from Stepan.

### 4. Ester Quaternary Ammonium Salts

A number of ester group containing quaternary ammonium salts, including those disclosed in EP 345842 A2 (Procter), EP 239910 (Procter) and US 4137180 (Lever) are known to be particularly useful as softening materials. These materials can be represented by generic formulae (IV) and (V) below.

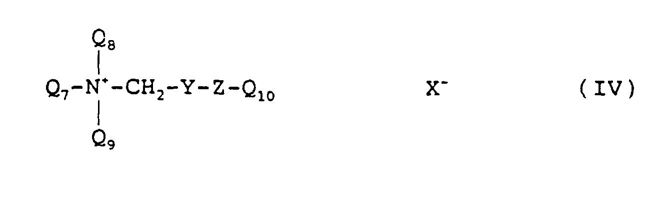

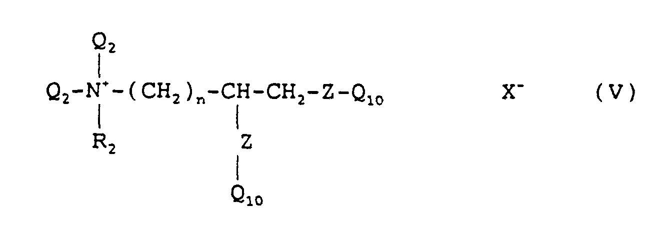

In formula (IV) Q₇ is a hydrocarbyl group containing 1 to 4 carbon atoms, Q₈ is (CH₂)ₙ-Z-Q₁₀ where n is an integer from 1 to 4 or -Q₁₀. Q₉ is an alkyl or hydroxyalkyl group of 1 to 4 carbon atoms, or is as defined for Q₈. Q₁₀ is a hydrocarbyl group containing from 12 to 22 carbon atoms and Y can be -CH(OH)-CH₂- or Q₆ as previously defined. Z can be -O-C(O)-O, -C(O)-O or -O-C(O)- and X⁻ is an anion.

In formula (V) the symbols Q₂, Q₁₀, Z and X⁻ have the meanings defined previously.

Examples of suitable materials based on formula IV are N,N-di(tallowyl-oxyethyl)-N,N-dimethyl ammonium chloride; N,N-di(2-tallowyloxy-2-oxo-ethyl)-N,N-dimethyl ammonium chloride; N,N-di(2-tallowyloxyethylcarbonyl oxyethyl)-N,N-dimethyl ammonium chloride; N-(2-tallowloxy-2-ethyl)-N-(2-tallowyl oxo-2-oxyethyl)-N,N-dimethyl ammonium chloride; N,N,N-tri(tallowyl-oxyethyl)-N-methyl ammonium chloride; N-(2-tallowyloxy-2-oxyethyl)-N-(tallowyl-N,N-dimethyl)-ammonium chloride. Tallowyl may be replaced with cocoyl, palmoyl, lauryl, oleyl, stearyl and palmityl groups. An illustrative example of a formula V material is 1,2-ditallowyloxy-3-trimethyl ammoniopropane chloride.

Examples of commercially available materials can be obtained under the trade name STEPANTEX VRH 90 (Stepan), AKYPOQUAT (Chem-Y) and as mixtures of mono and ditallow esters of 2,3-dihydroxy propane trimethyl ammonium chloride (HOECHST).

### 5. Quaternary Imidazolinium Salts

A further class of cationic softener materials is the imidazolinium salts of generic formula (VI). 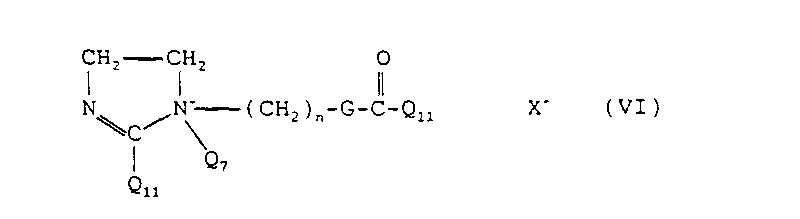 Wherein Q₁₁ is a hydrocarbyl group containing from 6 to 24 carbon atoms, G is -N(H)-, or -O-, or NQ₂, n is an integer between 1 and 4, and Q₇ is as defined above.

Preferred imidazolinium salts include 1-methyl-1-(tallowylamido) ethyl-2tallowyl-4,5 dihydro imidazolinium methosulphate and 1-methyl-1-(palmitoylamido) ethyl-2-octadecyl-4,5-dihydroimidazolinium chloride. Other useful imidazolinium materials are 2-heptadecyl-1-methyl-1-(2 stearylamido) ethyl imidazolinium chloride and 2-lauryl-1-hydroxyethyl-1-oleyl imidazolinium chloride. Also suitable are the imidazolinium fabric softening components of US 4127489. Representative commercially available materials are VARISOFT 475 (Sherex) and REWOQUAT W7500 (Rewo).

### 6. Primary Secondary and Tertiary Amines

Primary, secondary and tertiary amines of general formula (VII) are useful as softening agents.

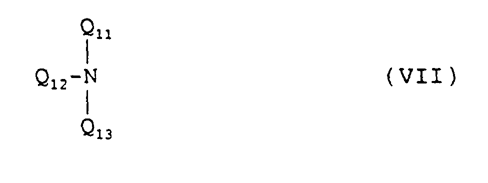

Wherein Q₁₁ is a hydrocarbyl group containing from 6 to 24 carbon atoms, Q₁₂ is hydrogen or a hydrocarbyl group containing from 1 to 22 carbon atoms and Q₁₃ can be hydrogen or Q₇. Preferably amines are protonated with hydrochloric acid, orthophosphoric acid or citric acid or any other similar acids for use in fabric conditioning compositions of this invention.

### 7. Alkoxylated Amines

Alkoxylated amines of general formula (VIII) are also useful as components of this invention.

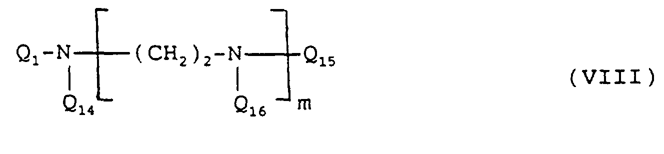

Wherein Q₁₄ is (C₂H₄O)ₓH, Q₁₅ is (C₂H₄O)_{y}h and Q₁₆ is (C₂H₄O)_{z}H and x+y is within the range 2 to 15 and x+y+z is within the range 3 to 15, m can be 0, 1 or 2 and Q₁ is as previously defined.

Examples of these materials are monotallow-dipolyethoxyamine containing from 2 to 30 ethylene oxide units, tallow N, N',N' tris (2-hydroxyethyl)-1,3 propylene diamine or C₁₀ to C₁₈ alkyl-N-bis(2-hydroxyethyl) amines. Examples of commercially available materials are available under the trade names ETHOMEEN and ETHODUOMEEN (Akzo).

### 8. Cyclic Amines

Other useful materials are dialkyl cyclic amines represented by formula (IX). 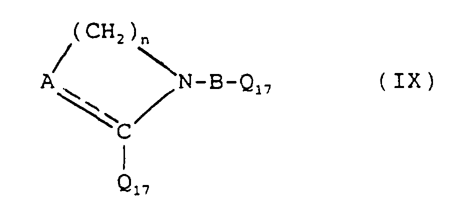 Wherein the groups Q₁₇ are independently selected from hydrocarbyl groups containing from 8 to 30 carbon atoms and A can be oxygen (-O-) or nitrogen (-N=) preferably nitrogen; B is selected from Q₆ as defined earlier or the group -Q₁₈-T-C(O)- where Q₁₈ is either Q₆ or (-C₂H₄O-)ₘ with m being an integer from 1 to 8 and T being selected from oxygen or NQ₁₃.

Illustrative materials are 12-stearyl oxyethyl-2-stearyl imidazoline, 1-stearyl oxylethyl-2-palmityl imidazoline, 1-stearyl oxyethyl myristyl imidazoline, 1-palmityl oxyethyl-2-palmityl imidazoline, 1-palmityl oxyethyl-2-myristyl imidazoline, 1-stearyl oxyethyl-2-tallow imidazoline, 1-myristyl oxyethyl-2-tallow imidazoline, 1-palmityl oxyethyl-2-tallow imidazoline, 1-coconut oxyethyl-2-coconut imidazoline, 1-tallow oxyethyl-2-tallow imidazoline and mixtures thereof. Also useful is stearyl hydroxyethyl imidazoline, available commercially as ALKAZINE (Alkaril), 1-tallow amido ethyl-2-tallow imidazoline and Methyl-1-tallow amidoethyl-2-tallow imidazoline.

Yet another class of suitable fabric softening materials are the condensation products formed from the reaction of fatty acids with a polyamine selected from the group consisting of hydroxyalkyl, alkylene diamines and dialkylenetriamines and mixtures thereof. Suitable materials are disclosed in EP-A-199382 (Procter). Preferred among these are mixtures of molecules of the generic formula X and corresponding salts obtained by partial protonation.

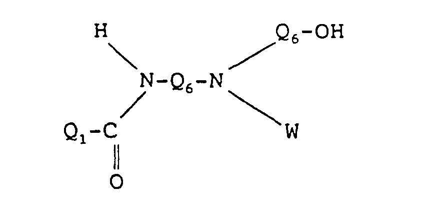

W is selected from hydrogen and the group -C(O)-Q₁ and other symbols are as previously defined. Commercially available materials of this class can be obtained from Sandoz Products as Ceranine HC39, HCA and HCPA.

### 9. Zwitterionic Fabric Softeners

Other useful ingredients of softening systems include zwitterionic quaternary ammonium compounds such as those disclosed in EP 332270 A2 (Unilever). Representative materials in this class are illustrated by general formula (XI) and (XII) 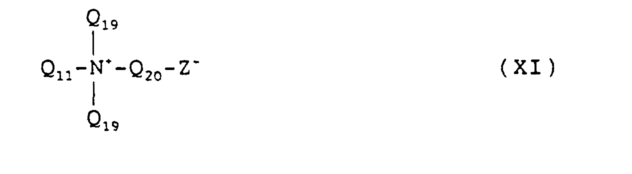 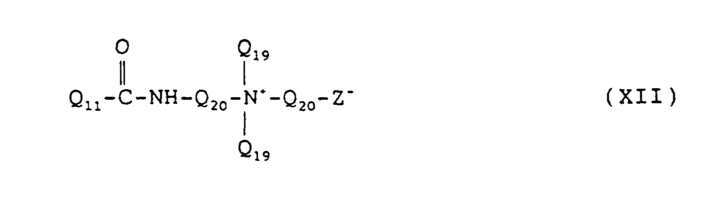 Wherein the groups Q₁₉ are selected independently from Q₇, Q₁₁ and Q₁₄; Q₂₀ is a divalent alkylene group containing 1 to 3 carbon atoms and may be interrupted by -O-, -CONH,-C(O)O-, etc; and Z' is an anionic water solubilising group (e.g. carboxy, sulphate, sulpho or phosphonium).

Examples of commercially available materials are the EMPIGEN CD and BS series (Albright Wilson) the REWOTERIC AM series (Rewo) and the Tegobetain F, H, L and N series (GOLDSCHMIDT).

### 10. Nonionic Ingredients

It is well known to blend nonionic materials with cationic, amphoteric or zwitterionic softening materials as a means of improving dispersion of the product in rinse waters and enhancing the fabric softening properties of the softener blend.

Suitable nonionic adjuncts include lanolin and lanolin derivatives, fatty acids containing from 10 to 18 carbon atoms, esters of fatty acids containing from 8 to 24 carbon atoms with monohydric alcohols containing from 1 to 3 carbon atoms, and polyhydric alcohols containing 2 to 8 carbon atoms such as sucrose, sorbitan, together with alkoxylated fatty acids, alcohols and lanolins containing an average of not more than 7 alkylene oxide groups per molecule. Suitable materials have been disclosed in EP-A-88520 (Unilever,), EP-A-122141 (Unilever), GB 2157728A (Unilever), GB 8410321 (Unilever), EP-A-159918 (Unilever), EP-A-159922 (Unilever) and EP-A-79746 (Procter).

Fabric softening compositions generally do not contain anionic detergent active nor bleach, nor detergency builder. It is desirable that the amounts (if any) of anionic detergent active, bleach and detergency builder are all less than the amount of the fabric softening agent.

A fabric softening composition which is intended to be added to rinse water may be in the form of a solid, a powder or tablet for instance, which disperses in the rinse water.

More commonly, a fabric softening composition for addition to rinse water is in the form of a liquid, and is an aqueous dispersion in water. Such a fabric softening composition may contain from 1% to 40% by weight of a fabric softening agent but may contain higher levels from 40% up to 80% or even 90% by weight in a very concentrated product. The composition will usually also contain water, which may provide the balance of the composition.

Liquid fabric softening compositions can be prepared by simply mixing the ingredients, including water, with agitation to disperse the water-insoluble ingredients.

Solid fabric conditioning articles which release a fabric softening agent in a tumble dryer can be designed for single usage or multiple usage.

One such article comprises a sponge material releasably enclosing a composition containing fabric softening agent and perfume so as to impart fabric softness and deodorancy during several drying cycles. This multi-use article can be made by filling a hollow sponge with the composition. In use, the composition melts and leaches out through the pores of the sponge to soften fabrics. Such a filled sponge can be used to treat several loads of fabrics in conventional dryers, and has the advantage that it can remain in the dryer after use and is not likely to be misplaced or lost.

Another article comprises a cloth or paper bag releasably enclosing such a composition and sealed with a hardened plug of the mixture. The action and heat of the dryer opens the bag and releases the composition to perform its softening and delivery of deodorant perfume function.

A highly preferred article comprises a composition containing the softening agent and deodorant perfume releasably impregnating a sheet of woven or non-woven cloth substrate. When such an article is placed in a tumble dryer the heat and tumbling action removes the fabric softening composition from the substrate and transfers it to the fabrics.

A solid product for use in a tumble dryer will generally contain fabric softening agent in an amount from 40% to 95% by weight of the product.

The amount of perfume incorporated in a fabric softening product will lie in the range from 0.01% to 10% by weight.

For fabric conditioning liquids containing less than 40% by weight of fabric softening agent, the amount of perfume is preferably 0.1% to 3% by weight, more preferably 0.1% to 1%, especially 0.1% to 0.3%.

The amount of perfume in very concentrated fabric conditioning liquids may lie in the broader range up to 10% by weight, preferably 2% to 8% by weight, more preferably 3% to 6% by weight.

The amount of perfume in products for use in a tumble dryer is preferably from 2% to 4% by weight of the product.

The deodorant effectiveness of a detergent or other composition which incorporates a perfume composition in accordance with this invention can be assessed by testing in accordance with Odour Reduction Value or Malodour Reduction Value tests as specified in the prior documents mentioned initially. These are based on the test devised by Whitehouse and Carter as published in "The proceedings of the Scientific Section of the Toilet Goods Association", No 48, December 1967 at pages 31-37 under the title "Evaluation of Deodorant Toilet Bars". For fabric conditioning compositions a suitable test procedure is a Malodour Reduction Value test based on that in US-A-4663068. It is derived from the original Whitehouse and Carter test.

The procedure is as follows:

Malodour Reduction Value test comprises the steps of:
(i) selecting pieces of cotton shirt fabric having an area of 20cm x 20cm or more;
(ii) washing the selected pieces of fabric in a front-loaded drum-type washing machine with an unperfumed washing powder: the composition of this powder is not critical but may be as follows:

| | Parts by weight |
|---|---|
| Sodium dodecylbenzene sulphonate | 9 |
| C13-15 alcohol 7EO | 4 |
| Sodium tripolyphosphate | 33 |
| Alkaline sodium silicate | 6 |
| Sodium carboxymethyl cellulose | 1 |
| Magnesium silicate | 1 |
| Ethylenediamine tetraacetic acid | 0.2 |
| Sodium sulphate | 25 |
| Water | 10.8 |

A suitable concentration in the wash liquor is 10.5 g/litre,
(iii) rinsing some of the washed pieces of fabric with water alone, and drying them to provide controls;
(iv) further pieces of fabric are rinsed using water to which is added a fabric softening composition consisting of:
6.25% di(hardened tallow) dimethyl ammonium chloride
0.05% preservative
0.4 % perfume under test
balance to 100% water

This composition is added to the rinse water in a ratio of 90ml of fabric softening composition to 24 litres of rinse water, i.e. 3.75 ml per litre of rinse water. Thereafter the fabric pieces are dried;
(v) stitching test and control pieces of fabric into clean polyester cotton shirts in the underarm region in accordance with a statistical design;
(vi) causing the shirts carrying the inserts to be worn by a panel of 40 Caucasian male subjects of age within a range from 20 to 55 years (the subjects being chosen from those who develop auxiliary body malodour that is not unusually strong and who do not develop a stronger body malodour in one axilla compared with the other);
(vii) assessing the body malodour of the fabric inserts after a period of five hours whereby three trained female assessors scored the olfactory intensity of malodour on a 0 to 5 scale, 0 representing no odour and 5 representing very strong malodour, the strength of the odour in each instance being related for purposes of comparison to standard odours produced by aqueous solutions of isovaleric acid at different concentrations according to the following table:

| Score | Odour Level | Conc. of aqueous isovaleric acid (ml/l) |
|---|---|---|
| 0 | No odour | 0 |
| 1 | Slight | 0.013 |
| 2 | Definite | 0.053 |
| 3 | Moderate | 0.22 |
| 4 | Strong | 0.87 |
| 5 | Very Strong | 3.57 |

(viii) calculating the average scores for test fabric pieces and control fabric pieces, and subtracting the average score for test pieces from the average score of the control pieces to arrive at the Malodour Reduction Value for the perfume composition under test.

### Examples 1 to 3

Perfume compositions in accordance with this invention were made and tested for deodorant action in a fabric softening composition, using the test for Malodour Reduction Value described above.

The perfume compositions are set out in the following tables. Where a material falls within one of the categories set forth above, this is indicated by the words "ketone", "alcohol", "acetate", "propionate" or "salicylate". Materials in the optional category of "ethers" are indicated as such. Materials in the other optional category of aldehydes of formula R⁷CHO and galaxolide are indicated by the abbreviation "aldh/glax".

These examples use a number of materials which are denoted by trade marks or trivial names. Some of these have been identified more fully above.

Others are:

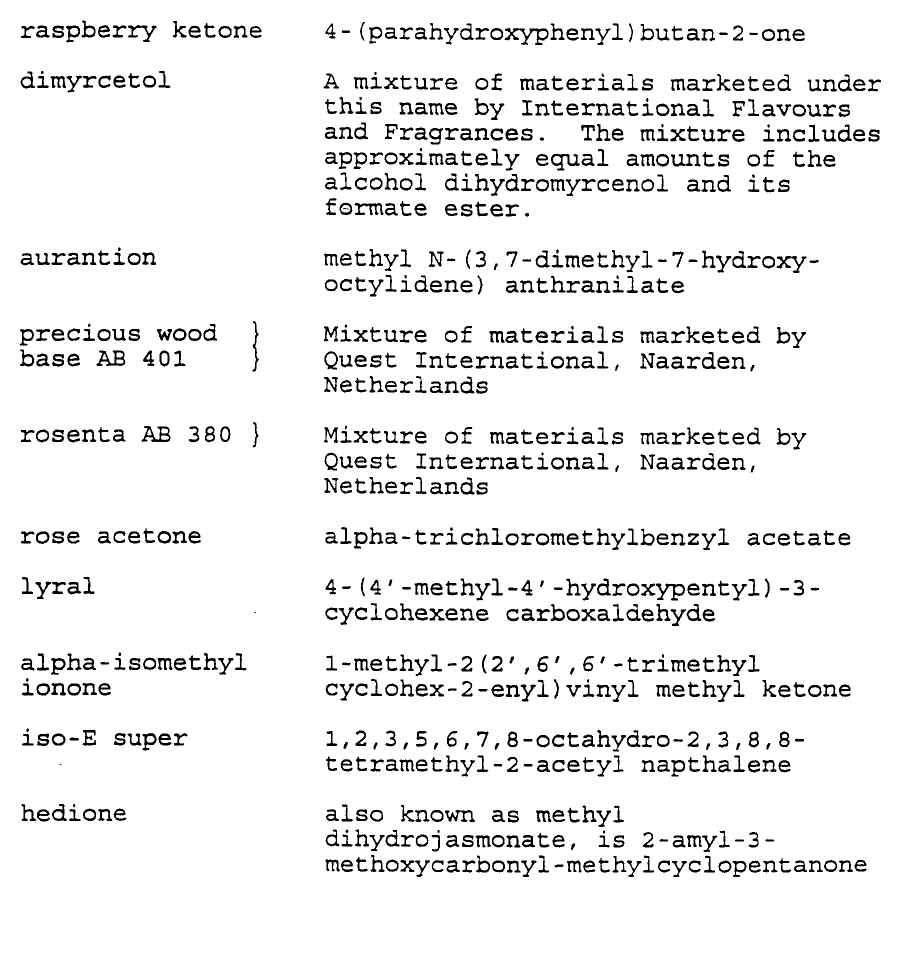

### EXAMPLE 1

| Ingredient | Category | % by weight |
|---|---|---|
| Tonalid | ketone | 3 |
| traseolide | ketone | 5 |
| dimyrcetol | alcohol (50%) | 2.5 |
| tetrahydrolinalol | alcohol | 6 |
| 9-decen-1-ol | alcohol | 0.5 |
| florocyclene | acetate | 2.2 |
| para tert butyl cyclohexyl acetate | acetate | 7 |
| dimethylbenzylcarbinylacetate | acetate | 2.5 |
| ortho tert butyl cyclohexyl acetate | acetate | 1.4 |
| benzyl salicylate | salicyl. | 6 |
| yara | ether | 0.1 |
| galaxolide | aldh/glax | 5 |
| hexyl cinnamic aldehyde | aldh/glax | 11 |
| lilial | aldh/glax | 10 |
| coumarin | | 2 |
| precious wood base AB 401 | | 3.8 |
| rose acetone | | 1 |
| acetyl cedrene | | 3.7 |
| benzyl acetate | | 3.5 |
| aurantion | | 2 |
| eugenol | | 0.5 |
| patchouli oil | | 1.8 |
| phenyl ethyl alcohol | | 7 |
| diethylphthalate | | 5 |
| alpha iso methyl ionone | | 5 |
| hedione | | 2.5 |

### EXAMPLE 2

| Ingredient | Category | % by weight |
|---|---|---|
| tonalid | ketone | 11 |
| traseolide | ketone | 5.5 |
| tetrahydrolinalol | alcohol | 6 |
| dihydromyrcenol | alcohol | 2 |
| florocyclene | acetate | 3 |
| para tert butyl cyclohexyl acetate | acetate | 6.6 |
| dimethylbenzylcarbinylacetate | acetate | 1 |
| ortho tert butyl cyclohexyl acetate | acetate | 1.5 |
| hexyl salicylate | salicyl. | 4.5 |
| benzyl salicylate | salicyl. | 2 |
| hexyl cinnamic aldehyde | aldh/glax | 16 |
| lilial | aldh/glax | 10 |
| aurantion | | 2.2 |
| linalol | | 2 |
| acetyl cedrene | | 4.5 |
| undecalactone gamma | | 0.2 |
| methyl cinnamate | | 0.7 |
| benzyl acetate | | 2.8 |
| lyral | | 0.2 |
| phenyl ethyl alcohol | | 6 |
| raspberry ketone | | 1 |
| alpha iso methyl ionone | | 3 |
| rosenta AB 380 | | 4.5 |
| iso-E super | | 1.8 |
| hedione | | 2 |

### EXAMPLE 3

| Ingredient | Category | % by weight |
|---|---|---|
| tonalid | ketone | 15 |
| traseolide | ketone | 6 |
| tetrahydrolinalol | alcohol | 5.5 |
| dihydromyrcenol | alcohol | 2 |
| florocyclene | acetate | 3 |
| para tert butyl cyclohexyl acetate | acetate | 5 |
| dimethylbenzylcarbinylacetate | acetate | 1 |
| ortho tert butyl cyclohexyl acetate | acetate | 1.6 |
| hexyl salicylate | salicyl. | 6.5 |
| benzyl salicylate | salicyl. | 2 |
| yara | ether | 0.5 |
| hexyl cinnamic aldehyde | aldh/glax | 15 |
| lilial | aldh/glax | 10 |
| coumarin | | 1.8 |
| aurantion | | 2 |
| acetyl cedrene | | 3.8 |
| methyl cinnamate | | 0.8 |
| benzyl acetate | | 2.5 |
| phenyl ethyl alcohol | | 7.5 |
| raspberry ketone | | 1 |
| rosenta AB 380 | | 5.5 |
| iso-E super | | 2 |

The results obtained in the Malodour Reduction Value test are set out in the following table.

| | Control | Average panel score | Malodour Reduction Value |
|---|---|---|---|
| Example 1 | 2.99 | 1.54 | 1.45 |
| Example 2 | 3.50 | 2.17 | 1.33 |
| Example 3 | 3.50 | 2.11 | 1.39 |

### Comparison of Odour Character

Perfumes according to Examples 1 to 3 above and one of the perfume compositions exemplified in US-A-4134838 were assessed for the character of their fragrances.

This assessment was carried out by a panel of thirty persons trained to recognise and discriminate between fragrance characteristics, e.g. floral, spicy etc. Each panelist was required to estimate the intensity of various characteristics in each perfume, relative to a standard material having the characteristic concerned. The panelists' scores were placed on a uniform scale and then averaged for each characteristic for each individual perfume. The scale was arranged to run from zero (denoting absence of the characteristic) to 2 (denoting the strongest individual characteristic which was "sharp" in the composition from US-A-4134838. On this scale the intensities of the individual characteristics in the standard materials were approximately 5.

The fragrance characteristics assessed included some which, although not necessarily unpleasant in themselves, are powerful and distinctive odours. Consequently, if these are perceptible in too great a degree in a perfume composition, they can render that perfume composition excessively distinctive and/or unattractive to a consumer or unsuitable for its intended application. Accordingly these characteristics should not dominate in a well balanced perfume.

The various fragrance characteristics which were assessed also included some which are generally considered attractive for the perfume of a fabric softening product.

The panel scores for individual characteristics in each perfume are set out in the following table.

The characteristic called "Mixed Florals" in the table is an overall score for eight individual characteristics which are the odours of individual flower species (carnation, hyacinth, jasmin, lilac, lily of the valley, narcissus, rose and violet).

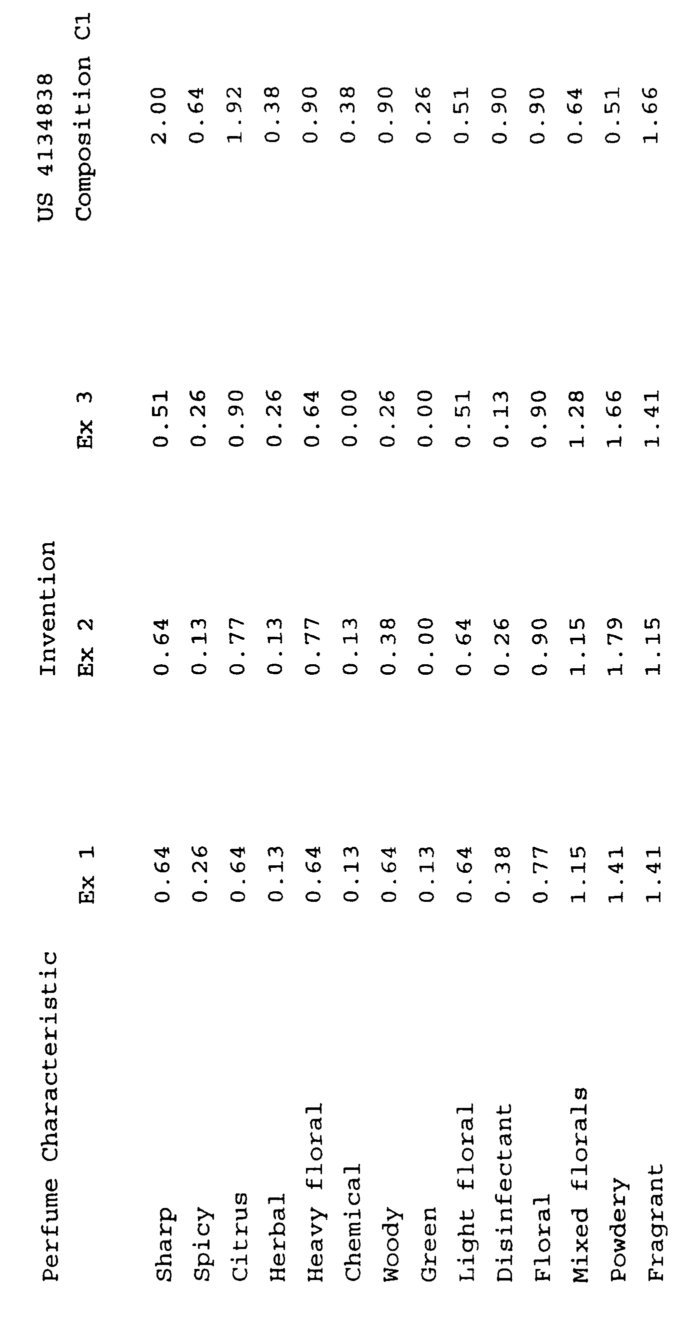

As can be seen from the above table, the perfume compositions exemplifying this invention have fairly high scores for characteristics which are attractive and appropriate in a perfume for a fabric softener, and lower scores for other characteristics, including undesirable or inappropriate characteristics such as "disinfectant", "citrus", "sharp", "spicy".

By contrast the characteristics of "sharp" and "citrus" were very strong in the composition C1 of the prior document.

## Claims

1. Use, as a deodorant, of a perfume composition in which at least 30% by weight of the composition is constituted by materials from the following categories:
at least 7% of one or more aromatic methyl ketones of general formula 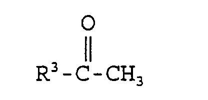 in which R³ is an aromatic group such that the molecular weight of the ketone is from 170 to 300;
at least 5% of one or more ingredients selected from: alcohols of general formula
R⁴OH
acetates of general formula
CH₃CO₂R⁵
propionates of general formula
C₂H₅CO₂R⁵
in which R⁴ is an aliphatic group, optionally containing not more than one olefinic double bond, and optionally bearing an aromatic substituent group, such that the molecular weight of the alcohol is in the range 130 to 180;
R⁵ is an aliphatic group optionally containing not more than one olefinic double bond, and optionally bearing an aromatic substituent group such that the molecular weight of the acetate or propionate is in the range 180 to 210;
at least 3% of one or more salicylates of general formula 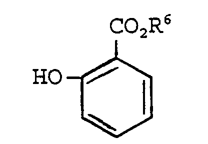 in which R⁶ is an aliphatic group, optionally containing not more than one olefinic double bond, and optionally bearing an aromatic substituent group, such that the molecular weight of the salicylate is in the range 190 to 230,
all the above percentages being by weight of the whole perfume composition,

2. Use according to claim 1 in which the materials present in the perfume composition include at least 4% of said alcohols R⁴OH and at least 5% of one or more said acetates or propionates.

3. Use according to claim 1 or claim 2 in which the perfume composition also includes at least 0.05% of one or more ethers of general formula
R¹OR²
in which the groups R¹ and R² are connected only through the ether oxygen atom, and are aliphatic or aromatic groups such that the ether has a molecular weight of 150 to 200.

4. Use according to claim 1 or claim 2 or claim 3 in which the materials present in the perfume composition include at least 2% by weight of one or more compounds which are either aldehydes of formula R⁷CHO in which R⁷ is an aliphatic group, optionally containing not more than one olefinic double bond, and optionally bearing an aromatic substituent group, such that the molecular weight of the aldehyde is in the range 180 to 220; or 1,3,4,6,7,8-hexahydro-4,6,6,7,8,8-hexamethyl cyclopenta-2-benzopyran.

5. Use according to any one of claims 1 to 4 wherein the perfume composition contains
at least 4% of said alcohols, which do not include any alcohol which is also an ester, and
at least 7% of said ketones, which do not include any ketone which is also an ester or an alcohol.

6. Use according to claim 5 wherein the specified quantities of said ketones, alcohols, acetates or propionates, and salicylates are provided by materials which are not aldehydes.

7. A fabric conditioning product comprising
(i) a fabric softening agent and
(ii) a perfume composition in which at least 30% by weight of the perfume composition is constituted by materials from the following categories:
at least 7% of one or more aromatic methyl ketones of general formula 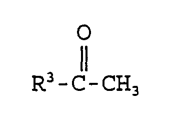 in which R³ is an aromatic group such that the molecular weight of the ketone is from 170 to 300;
at least 5% of one or more ingredients selected from: alcohols of general formula
R⁴OH
acetates of general formula
CH₃CO₂R⁵
propionates of general formula
C₂H₅CO₂R⁵
in which R⁴ is an aliphatic group, optionally containing not more than one olefinic double bond, and optionally bearing an aromatic substituent group, such that the molecular weight of the alcohol is in the range 130 to 180;
R⁵ is an aliphatic group optionally containing not more than one olefinic double bond, and optionally bearing an aromatic substituent group such that the molecular weight of the acetate or propionate is in the range 180 to 210;
at least 3% of one or more salicylates of general formula 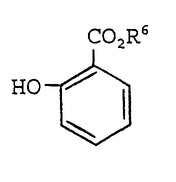 in which R⁶ is an aliphatic group, optionally containing not more than one olefinic double bond, and optionally bearing an aromatic substituent group, such that the molecular weight of the salicylate is in the range 190 to 230,
all the above percentages being by weight of the whole perfume composition,

8. A fabric conditioning product according to claim 7 in which the materials present in the perfume composition include at least 4% of said alcohols R⁴OH and at least 5% of one or more said acetates or propionates.

9. A fabric conditioning product according to claim 7 or claim 8 in which the perfume composition also includes at least 0.05% of one or more ethers of general formula
R¹OR²
in which the groups R¹ and R² are connected only through the ether oxygen atom, and are aliphatic or aromatic groups such that the ether has a molecular weight of 150 to 200.

10. A fabric conditioning product according to claim 7, claim 8, or claim 9 in which the materials present in the perfume composition include at least 2% by weight of one or more compounds which are either aldehydes of formula R⁷CHO in which R⁷ is an aliphatic group, optionally containing not more than one olefinic double bond, and optionally bearing an aromatic substituent group, such that the molecular weight of the aldehyde is in the range 180 to 220; or 1,3,4,6,7,8-hexahydro-4,6,6,7,8,8-hexamethyl cyclopenta-2-benzopyran.

11. A fabric conditioning product according to any one of claims 7 to 10 wherein the perfume composition contains
at least 4% of said alcohols, which do not include any alcohol which is also an ester, and
at least 7% of said ketones, which do not include any ketone which is also an ester or an alcohol.

12. A fabric conditioning product according to claim 11 wherein the specified quantities of said ketones, alcohols, acetates or propionates, and salicylates are provided by materials which are not aldehydes.

13. A fabric conditioning product according to any one of claims 7 to 12 wherein the perfume composition comprises
from 7% to 50% by weight of the perfume composition of one or more said ketones, not including any ketone which is also an alcohol,
from 4% to 50% by weight of the perfume composition of one or more said alcohols, not including any alcohol which is also an ester,
from 4% to 40% by weight of the perfume composition of an ester selected from the group consisting of said acetates and propionates,
from 3% to 60% by weight of the perfume composition or one or more said salicylates, and
from 0.1% to 20% by weight of the perfume composition of one or more ethers of general formula
R¹OR²
in which the groups R¹ and R² are connected only through the ether oxygen atom, and are aliphatic or aromatic groups such that the ether has a molecular weight of 150 to 200.

14. A fabric conditioning product according to any one of claims 7 to 13 wherein the fabric softening agent comprises an organic quaternary nitrogen compound including at least one carbon chain of 60 to 30 carbon atoms.

15. A method of perfuming fabrics comprising laundering the fabrics and them rinsing the fabrics in an aqueous rinse liquor containing a composition as defined in any one of claims 7 to 14.

## Patentansprüche

1. Verwendung einer Parfumzusammensetzung als Deodorant, wobei zumindest 30 Gew.-% der Zusammensetzung aus Materialien aus den folgenden Kategorien bestehen:
zumindest 7 % eines oder mehrerer Methylketone der allgemeinen Formel 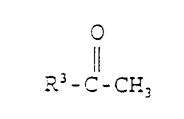 worin R³ eine solche aromatische Gruppe ist, dass das Molekulargewicht des Ketons 170 bis 300 beträgt;
zumindest 5 % eines oder mehrerer Bestandteile, ausgewählt aus:
Alkoholen der allgemeinen Formel
R⁴OH,
Acetaten der allgemeinen Formel
CH₃CO₂R⁵,
Propionaten der allgemeinen Formel
C₂H₅CO₂R⁵,
worin R⁴ eine aliphatische Gruppe ist, die gegebenenfalls nicht mehr als eine olefinische Doppelbindung enthält und gegebenenfalls eine solche aromatische Substituentengruppe aufweist, dass das Molekulargewicht des Alkohols im Bereich von 130 bis 180 liegt;
R⁵ eine aliphatische Gruppe ist, die gegebenenfalls nicht mehr als eine olefinische Doppelbindung enthält und gegebenenfalls eine solche aromatische Substituentengruppe aufweist, dass das Molekulargewicht des Acetats oder Propionats im Bereich von 180 bis 210 liegt;
zumindest 3 % eines oder mehrerer Salicylate der allgemeinen Formel 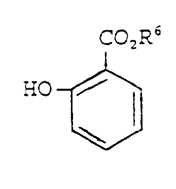 worin R⁶ eine aliphatische Gruppe ist, die gegebenenfalls nicht mehr als eine olefinische Doppelbindung enthält, und gegebenenfalls eine solche aromatische Substituentengruppe aufweist, dass das Molekulargewicht des Salicylats im Bereich von 190 bis 230 liegt,
wobei alle obigen Prozentsätze Gewichtsprozent, bezogen auf die gesamte Parfumzusammensetzung, sind.

2. Verwendung nach Anspruch 2, wobei die in der Parfumzusammensetzung enthaltenen Materialien zumindest 4 % der Alkohole R⁴OH und zumindest 5 % eines oder mehrerer der Acetate oder Propionate umfassen.

3. Verwendung nach Anspruch 1 oder 2, wobei die Parfumzusammensetzung auch zumindest 0,05 % eines oder mehrerer Ether der allgemeinen Formel
R¹OR²
enthält, worin die Gruppen R¹ und R² nur über das Ether-Sauerstoffatom verbunden sind und solche aliphatische oder aromatische Gruppen sind, dass der Ether ein Molekulargewicht von 150 bis 200 aufweist.

4. Verwendung nach Anspruch 1, 2 oder 3, wobei die in der Parfumzusammensetzung enthaltenen Materialien zumindest 2 Gew.-% einer oder mehrerer Verbindungen umfassen, die entweder Aldehyde der Formel R⁷OH sind, worin R⁷ eine aliphatische Gruppe ist, die gegebenenfalls nicht mehr als eine olefinische Doppelbindung enthält und gegebenenfalls eine solche aromatische Substituentengruppe aufweist, dass das Molekulargewicht des Aldehyds im Bereich von 180 bis 220 liegt; oder 1,3,4,6,7,8-Hexahydro-4,6,6,7,8,8-hexamethylcyclopenta-2-benzopyran ist.

5. Verwendung nach einem der Ansprüche 1 bis 4, worin die Parfumzusammensetzung Folgendes enthält:
zumindest 4 % der Alkohole, wobei kein Alkohol inkludiert ist, der auch ein Ester ist, und
zumindest 7 % der Ketone, wobei kein Keton inkludiert ist, das auch ein Ester oder ein Alkohol ist.

6. Verwendung nach Anspruch 5, worin die angegebenen Mengen der Ketone, Alkohole, Acetate oder Propionate und der Salicylate Materialien umfassen, die keine Aldehyde sind.

7. Gewebekonditionierungsprodukt, umfassend:
(i) einen Gewebe-Weichmacher und
(ii) eine Parfumzusammensetzung, wobei zumindest 30 Cew.-% der Parfumzusammensetzung aus Materialien aus den folgenden Kategorien bestehen:
zumindest 7 % eines oder mehrerer Methylketone der allgemeinen Formel 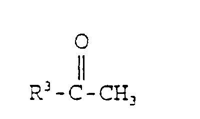 worin R³ eine solche aromatische Gruppe ist, dass das Molekulargewicht des Ketons 170 bis 300 beträgt;
zumindest 5 % eines oder mehrerer Bestandteile, ausgewählt aus:
Alkoholen der allgemeinen Formel
R⁴OH,
Acetaten der allgemeinen Formel
CH₃CO₂R⁵,
Propionaten der allgemeinen Formel
C₂H₅CO₂R⁵,
worin R⁴ eine aliphatische Gruppe ist, die gegebenenfalls nicht mehr als eine olefinische Doppelbindung enthält und gegebenenfalls eine solche aromatische Substituentengruppe aufweist, dass das Molekulargewicht des Alkohols im Bereich von 130 bis 180 liegt;
R⁵ eine aliphatische Gruppe ist, die gegebenenfalls nicht mehr als eine olefinische Doppelbindung enthält und gegebenenfalls eine solche aromatische Substituentengruppe aufweist, dass das Molekulargewicht des Acetats oder Propionats im Bereich von 180 bis 210 liegt;
zumindest 3 % eines oder mehrerer Salicylate der allgemeinen Formel 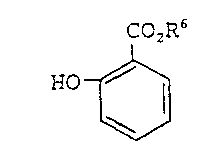 worin R⁶ eine aliphatische Gruppe ist, die gegebenenfalls nicht mehr als eine olefinische Doppelbindung enthält, und gegebenenfalls eine solche aromatische Substituentengruppe aufweist, dass das Molekulargewicht des Salicylats im Bereich von 190 bis 230 liegt,
wobei alle obigen Prozentsätze Gewichtsprozent, bezogen auf die gesamte Parfumzusammensetzung, sind.

8. Gewebekonditionierungsprodukt nach Anspruch 7, worin die in der Parfumzusammensetzung enthaltenen Materialien zumindest 4 % der Alkohole R⁴OH und zumindest 5 % eines oder mehrerer der Acetate oder Propionate umfassen.

9. Gewebekonditionierungsprodukt nach Anspruch 7 oder 8, wobei die Parfumzusammensetzung auch zumindest 0,05 % eines oder mehrerer Ether der allgemeinen Formel
R¹OR²
enthält, worin die Gruppen R¹ und R² nur über das Ether-Sauerstoffatom verbunden sind und solche aliphatische oder aromatische Gruppen sind, dass der Ether ein Molekulargewicht von 150 bis 200 aufweist.

10. Verwendung nach Anspruch 7, 8 oder 9, wobei die in der Parfumzusammensetzung enthaltenen Materialien zumindest 2 Gew.-% einer oder mehrerer Verbindungen umfassen, die entweder Aldehyde der Formel R⁷OH sind, worin R⁷ eine aliphatische Gruppe ist, die gegebenenfalls nicht mehr als eine olefinische Doppelbindung enthält und gegebenenfalls eine solche aromatische Substituentengruppe aufweist, dass das Molekulargewicht des Aldehyds im Bereich von 180 bis 220 liegt; oder 1,3,4,6,7,8-Hexahydro-4,6,6,7,8,8-hexamethylcyclopenta-2-benzopyran ist.

11. Gewebekonditionierungsprodukt nach einem der Ansprüche 7 bis 10, worin die Parfumzusammensetzung Folgendes enthält:
zumindest 4 % der Alkohole, wobei kein Alkohol inkludiert ist, der auch ein Ester ist, und
zumindest 7 % der Ketone, wobei kein Keton inkludiert ist, das auch ein Ester oder ein Alkohol ist.

12. Gewebekonditionierungsprodukt nach Anspruch 11, worin die angegebenen Mengen der Ketone, Alkohole, Acetate oder Propionate und der Salicylate Materialien umfassen, die keine Aldehyde sind.

13. Gewebekonditionierungsprodukt nach einem der Ansprüche 7 bis 12, worin die Parfumzusammensetzung Folgendes umfasst:
7 bis 50 Gew.-% der Parfumzusammensetzung eines oder mehrerer der Ketone, wobei kein Keton inkludiert ist, das auch ein Alkohol ist,
4 bis 50 Gew.-% der Parfumzusammensetzung eines oder mehrerer der Alkohole, wobei kein Alkohol inkludiert ist, der auch ein Ester ist,
4 bis 40 Gew.-% der Parfumzusammensetzung eines Ester, ausgewählt aus der aus den Acetaten und Propionaten bestehenden Gruppe,
3 bis 60 Gew.-% der Parfumzusammensetzung eines oder mehrerer der Salicylate, und
0,1 bis 20 Gew.-% der Parfumzusammensetzung eines oder mehrerer Ether der allgemeinen Formel
R¹OR²,
worin die Gruppen R¹ und R² nur über das Ether-Sauerstoffatom verbunden sind und solche aliphatische oder aromatische Gruppen sind, dass der Ether ein Molekulargewicht von 1 50 bis 200 aufweist.

14. Gewebekonditionierungsprodukt nach einem der Ansprüche 7 bis 13, worin der Gewebe-Weichmacher eine organische quaternäre Stickstoffverbindung umfasst, die zumindest eine Kohlenstoffkette mit 60 bis 30 Kohlenstoffatomen enthält.

15. Verfahren zum Parfumieren von Geweben, umfassend das Waschen der Gewebe und das anschließende Spülen der Gewebe in einer wässrigen Spüllösung, die eine Zusammensetzung nach einem der Ansprüche 7 bis 14 enthält.

## Revendications

1. Utilisation, comme déodorant, d'une composition de parfum où au moins 30% en poids de la composition est constitué de matières dans les catégories suivantes:
au moins 7% d'une ou plusieurs méthyl cétones aromatiques de la formule générale 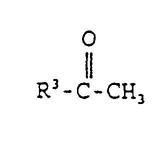 dans laquelle R³ est un groupe aromatique tel que le poids moléculaire de la cétone soit de 170 à 300;
au moins 5% d'un ou plusieurs ingrédients sélectionnés parmi:
alcools de la formule générale
R⁴OH
acétates de la formule générale
CH₃CO₂R⁵
propionates de la formule générale
C₂H₅CO₂R⁵
où R⁴ est un groupe aliphatique, ne contenant facultativement pas plus d'une double liaison oléfinique, et portant facultativement un groupe substituant aromatique, de façon que le poids moléculaire de l'alcool soit dans la gamme de 130 à 180;
R⁵ est un groupe aliphatique ne contenant facultativement pas plus d'une double liaison oléfinique, et portant facultativement un groupe substituant aromatique tel que le poids moléculaire de l'acétate ou du propionate soit dans la gamme de 180 à 210;
au moins 3% d'un ou plusieurs salicylates de la formule générale 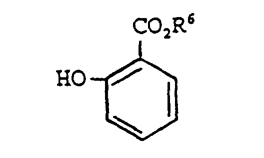 où R⁶ est un groupe aliphatique, ne contenant facultativement pas plus d'une double liaison oléfinique, et portant facultativement un groupe substituant aromatique de façon que le poids moléculaire du salicylate soit dans la gamme de 190 à 230,
tous les pourcentages ci-dessus étant en poids de la composition de parfum complète.

2. Utilisation selon la revendication 1, dans laquelle les matières présentes dans la composition de parfum comprennent au moins 4% desdits alcools R⁴OH et au moins 5% d'un ou plusieurs desdits acétates ou propionates.

3. Utilisation selon la revendication 1 ou la revendication 2 dans laquelle la composition de parfum contient également au moins 0,05% d'un ou plusieurs éthers de la formule générale
R¹OR²
où les groupes R¹ et R² sont connectés uniquement par l'atome d'oxygène de l'éther, et sont des groupes aliphatiques ou aromatiques de façon que l'éther ait un poids moléculaire de 150 à 200.

4. Utilisation selon la revendication 1 ou la revendication 2 ou la revendication 3, dans laquelle les matières présentes dans la composition de parfum comprennent au moins 2% en poids d'un ou plusieurs composés qui sont soit des aldéhydes de formule R⁷CHO où R⁷ est un groupe aliphatique ne contenant facultativement pas plus d'une double liaison oléfinique, et portant facultativement un groupe substituant aromatique de façon que le poids moléculaire de l'aldéhyde soit dans la gamme de 180 à 220; ou du 1,3,4,6,7,8-hexahydro-4,6,6,7,8,8-hexaméthyl cyclopenta-2-benzopyrane.

5. Utilisation selon l'une quelconque des revendications 1 à 4 où la composition de parfum contient
au moins 4% desdits alcools, qui ne comprennent pas d'alcool qui est également un ester, et
au moins 7% desdites cétones, qui ne comprennent pas une cétone qui est également un ester ou un alcool.

6. Utilisation selon la revendication 5, où les quantités spécifiées desdites cétones, desdits alcools, desdits acétates ou propionates, et desdits salicylates sont formées par des matières qui ne sont pas des aldéhydes.

7. Produit de conditionnement des étoffes comprenant
(i) un agent assouplissant les étoffes et
(ii) une composition de parfum où au moins 30% en poids de la composition de parfum est constitué par des matières des catégories suivantes:
au moins 7% d'un ou plusieurs méthyl cétones aromatiques de la formule générale 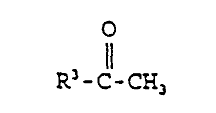 où R³ est un groupe aromatique tel que le poids moléculaire de l'acétone soit de 170 à 300;
Au moins 5% d'un ou plusieurs ingrédients sélectionnés parmi
alcools de la formule générale
R⁴OH
acétates de la formule générale
CH₃CO₂R⁵
propionates de la formule générale
C₂H₅CO₂R⁵
où R⁴ est un groupe aliphatique ne contenant facultativement pas plus d'une double liaison oléfinique et portant facultativement un groupe substituant aromatique de façon que le poids moléculaire de l'alcool soit dans la gamme de 130 à 180;
R⁵ est un groupe aliphatique ne contenant facultativement pas plus d'une double liaison oléfinique, et portant facultativement un groupe substituant aromatique tel que le poids moléculaire de l'acétate ou du propionate soit dans la gamme de 180 à 210;
au moins 3% d'un ou plusieurs salicylates de la formule générale 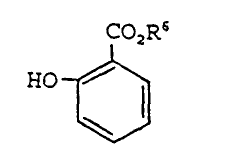 où R⁶ est un groupe aliphatique ne contenant facultativement pas plus d'une double liaison oléfinique, et portant facultativement un groupe substituant aromatique, de façon que le poids moléculaire du salicylate soit dans la gamme de 190 à 230,
tous les pourcentages ci-dessus étant en poids de la composition de parfum entière.

8. Produit de conditionnement d'une étoffe selon la revendication 7, où les matières présentes dans la composition de parfum comprennent au moins 4% desdits alcools R⁴OH et au moins 5% d'un ou plusieurs desdits acétates ou propionates.

9. Produit de conditionnement d'une étoffe selon la revendication 7 ou la revendication 8, où la composition de parfum contient également au moins 0,05% d'un ou plusieurs éthers de la formule générale
R¹OR²
où les groupes R¹ et R² sont connectés uniquement par l'atome d'oxygène de l'éther, et sont des groupes aliphatiques ou aromatiques de façon que l'éther ait un poids moléculaire de 150 à 200.

10. Produit de conditionnement d'une étoffe selon la revendication 7, la revendication 8, ou la revendication 9, où les matières présentes dans la composition de parfum comprennent au moins 2% en poids d'un ou plusieurs composés qui sont soit des aldéhydes de la formule R⁷CHO où R⁷ est un groupe aliphatique, ne contenant facultativement pas plus d'une double liaison oléfinique et portant facultativement un groupe substituant aromatique de façon que le poids moléculaire de l'aldéhyde soit dans la gamme de 180 à 220; ou
du 1,3,4,6,7,8-hexahydro-4,6,6,7,8,8-hexaméthyl cyclopenta-2-benzopyrane.

11. Produit de conditionnement d'une étoffe selon l'une quelconque des revendications 7 à 10, où la composition de parfum contient
au moins 4% desdits alcools, qui ne comprennent pas d'alcool qui est également un ester, et
au moins 7% desdites cétones qui ne comprennent pas de cétone qui est également un ester ou un alcool.

12. Produit de conditionnement d'une étoffe selon la revendication 11 où les quantités spécifiées desdites cétones, desdits alcools, desdits acétates ou propionates et desdits salicylates sont formées par des matières qui ne sont pas des aldéhydes.

13. Produit de conditionnement d'une étoffe selon l'une quelconque des revendications 7 à 12 où la composition de parfum comprend
de 7 à 50% en poids de la composition de parfum d'une ou plusieurs desdites cétones, ne comprenant pas de cétone qui est également un alcool,
de 4% à 50% en poids de la composition de parfum d'un ou plusieurs desdits alcools, ne comprenant pas d'alcool qui est également un ester,
de 4% à 40% en poids de la composition de parfum d'un ester sélectionné dans le groupe consistant en lesdits acétates et propionates,
de 3% à 60% en poids de la composition de parfum d'un ou plusieurs desdits salicylates, et
de 0,1% à 20% en poids de la composition de parfum d'un ou plusieurs éthers de la formule générale
R¹OR²
où les groupes R¹ et R² sont connectés uniquement par l'atome d'oxygène de l'éther, et sont des groupes aliphatiques ou aromatiques de façon que l'éther ait un poids moléculaire de 150 à 200.

14. Produit de conditionnement d'une étoffe selon l'une quelconque des revendications 7 à 13 où l'agent assouplissant l'étoffe comprend un composé d'azote quaternaire organique comprenant au moins une chaîne de carbone de 60 à 30 atomes de carbone.

15. Méthode pour parfumer les étoffes comprenant le blanchissage des étoffes puis leur rinçage dans une liqueur aqueuse de rinçage contenant une composition telle que définie dans l'une quelconque des revendications 7 à 14.
